# EUROPEAN PATENT APPLICATION

(11) **EP 3 150 157 A1**
(43) Date of publication of application: **05.04.2017**
(21) Application number: 15800416.8
(22) Date of filing: 11.05.2015
(51) Int. Cl.: A61B 18/12, A61B 18/14, A61N 1/06, A61N 1/08

(54) **DEVICE AND METHOD FOR DETECTING POSITION OF ELECTRODE INSERTED INTO HUMAN BODY**

(30) Priority: 28.05.2014 KR 20140064323
(71) Applicant: Taewoong Medical Co., Ltd., Gimpo-si, Gyeonggi-do 415-873 (KR); Starmed Co., Ltd., Ilsandong-gu, Goyang-si Gyeonggi-do 410-722 (KR); Samsung Life Public Welfare Foundation, Seoul 140-893 (KR)
(72) Inventor: SHIN, Kyong Min, Yangpyeong-gun Gyeonggi-do 476-801 (KR); SHIN, Kyung Hoon, Gimpo-si Gyeonggi-do 415-738 (KR); KIM, Dong Un, Gimpo-si Gyeonggi-do 415-090 (KR); LEE, Jun Hyok, Gimpo-si Gyeonggi-do 415-060 (KR); RHIM, Hyun Chul, Seoul 138-777 (KR); LEE, Min Woo, Seoul 138-910 (KR)
(74) Representative: Stöckeler, Ferdinand
(86) International application number: PCT/KR2015/004671
(87) International publication number: WO 2015/182895

(57) **Abstract**

The present invention relates to a device and a method for detecting a position of an electrode inserted into a human body that predict a path through a position sensor in a handle part before the electrode is inserted into the human body and display the path. The device for detecting the position of an electrode inserted into a human body according to the present invention includes: a position signal generator generating a position signal; an electrode needle having an electrode-side position sensor that is provided in an interior of a lance needle thereof and receives the position signal to generate first position information; and a handle connected to the electrode needle and having a handle-side position sensor that is included therein and receives the position signal to generate second position information.

## Description

### [Technical Field]

The present invention relates to a device and a method for detecting a position of an electrode inserted into a human body that predict a path through a position sensor in a handle part before the electrode is inserted and display the path in order to accurately place an electrode needle on a lesion through a position sensor in the electrode needle after the electrode is inserted into the human body in an electrode device for radio frequency ablation.

### [Background Art]

In general, when a cancer tissue or the like is generated in a body organ, a treatment therefor is performed by a surgical operation, however, since the surgical operation generally needs to resect a site of a lesion of a body, there are problems that the resected site is very large to cause a huge scar, a long convalescence period is required, etc.

Accordingly, non-surgical methods such as transarterial chemoembolization therapy, percutaneous ethanol injection therapy, systemic chemotherapy, local thermotherapy, etc., are used for the treatment, and among them, the local thermotherapy is known to be most effective in terms of short treatment period or improvement of long-term survival rate.

The local thermotherapy includes radio frequency ablation, microwave cauterization, laser cauterization, etc. and among them, the radio frequency ablation has been most effectively used. Here, the radio frequency ablation is a treatment method of, when a cancer tissue is generated in a body organ, for example, a liver, cauterizing and necrotizing only the cancer tissue with high frequency heat, without resecting the liver.

To this end, the existing electrode device for radio frequency ablation generally attaches a ground pad on epidermis of a patient as a passive electrode, inserts a needle-shaped electrode into a site of a lesion as an active electrode, and electrically connects the ground pad and the needle-shaped electrode to a radio frequency generator.

Therefore, when applying power to the radio frequency generator, a transfer path of a current from the electrode toward the ground pad is formed, and in the transfer process, frictional energy caused by vibration of ions increases a temperature of a tissue, thereby inducing coagulation and necrosis of the tissue of the site of the lesion.

However, in the case of the existing electrode device for radio frequency ablation operated as described above, since an operator inserts a medical needle such as an ablation needle, a biopsy needle or the like, by confirming the site of the lesion with his/her eye without directly resecting the human body, whether the electrode needle is accurately inserted into the lesion may not be confirmed.

Further, whether the electrode needle is accurately inserted into the lesion may not be confirmed when the electrode needle is bent when being inserted into the site of the lesion.

### [Disclosure]

### [Technical Problem]

An object of the present invention to solve the above described problems is to provide a device and a method for detecting a position of an electrode inserted into a human body that predict a path through a position sensor in a handle part before the electrode is inserted and display the path in order to accurately place an electrode needle on a lesion through a position sensor in the electrode needle after the electrode is inserted into the human body during ultrasonic image diagnosis, computerized tomography (CT), or magnetic resonance imaging (MRI) guide radio frequency ablation (RFA) operation in an electrode device for radio frequency ablation that cauterizes and necrotizes a site of a lesion, such as a tumor tissue of a body organ, etc., by heating the site of the lesion with high frequency.

### [Technical Solution]

According to the present invention, a device for detecting the position of an electrode inserted into a human body includes: a position signal generator generating a position signal; an electrode needle having an electrode-side position sensor that is provided in an interior of a lance needle thereof and receives the position signal to generate first position information; and a handle connected to the electrode needle and having a handle-side position sensor that is included therein and receives the position signal to generate second position information.

The electrode needle may include a body inserted into a tissue of a site of a lesion, and an active electrode and a passive electrode that are wound around the body.

The body may be formed in a needle shape that is long and fine, or in a cylindrical shape that is long and fine, the body formed in the needle shape may have an end that is sharp so as to be easily inserted into the tissue of the site of the lesion and the other end that is connected to the handle, and the body formed in the cylindrical shape may be connected to an end portion of a moving wire when being applied to a catheter.

The device may further include a radio frequency generator generating a high frequency alternating current and supplying the high frequency alternating current to the electrode needle by selective connection of the active electrode or the passive electrode to a positive terminal and a negative terminal.

The active electrode and the passive electrode may radiate the high frequency alternating current generated from the radio frequency generator to the electrode needle, and be each inclinedly wound in a spiral direction toward a rear end side from a front end portion of an outer circumferential surface of the body in two or more plural times at the same lead angle while being in parallel with each other.

The handle may be a part gripped by an operator when intending to use the electrode needle, be disposed at a rear end of the electrode needle, and have an electrode wire extended to be long to the radio frequency generator for electrically connecting the electrode needle and the radio frequency generator, and a cooling pipe connected thereto for supplying and collecting cooling water to circulate the cooling water.

The device may further include an electrode needle image processor receiving the first position information from the electrode-side position sensor provided in the electrode needle, and the second position information from the handle-side position sensor provided in the handle to display an insertion path on a screen based on the first position information and the second position information when the electrode needle is inserted into the human body.

The electrode needle image processor may display an insertion position range approachable by the electrode needle around a site of a lesion based on the second position information received from the handle-side position sensor, and display the insertion path through which the electrode needle inserted into the human body will pass, based on the first position information received from the electrode-side position sensor when the electrode needle is inserted into the human body within the insertion position range.

The electrode needle image processor may recognize, based on the first position information and the second position information, that the electrode needle is inserted while being bent in the human body when the insertion path is not a straight line , and calculate an angle and a radius of curvature at which the electrode needle is bent based on a distance by which the first position information is spaced apart from a straight line on the basis of the second position information and display the calculated result on the screen.

The electrode needle image processor may display the insertion path through which the electrode needle is inserted into the human body on the screen by expanding a signal reception range to a signal reception range based on the second position information received from the handle-side position sensor, in addition to a signal reception range based on the first position information received from the electrode-side position sensor, and accumulating the first position information and the second position information.

According to the present invention, a method for detecting a position of an electrode inserted into a human body includes: (a) generating, by a position signal generator, a position signal; (b) receiving, by a handle-side position sensor, the position signal to generate second position information; (c) receiving, by an electrode needle image processor, the second position information to display an insertion position range approachable by an electrode needle around a site of a lesion; (d) receiving, by an electrode-side position sensor, the position signal to generate first position information; (e) receiving, by the electrode needle image processor, the first position information to display a position in the human body where the electrode needle is inserted within the insertion position range; and (f) displaying, by the electrode needle image processor, an insertion path through which the electrode needle inserted into the human body will pass, on a screen based on the first position information and the second position information.

The method may further include: (g) generating, by a radio frequency generator, a high frequency alternating current and supplying the high frequency alternating current to the electrode needle by selective connection of an active electrode or a passive electrode to a positive terminal and a negative terminal.

In the (f) displaying, the electrode needle image processor may display the insertion path in a straight line or a curved line on the screen based on the first position information and the second position information.

In the (f) displaying, the electrode needle image processor may recognize, based on the first position information and the second position information, that the electrode needle is inserted while being bent in the human body when the insertion path is not a straight line , and calculate an angle and a radius of curvature at which the electrode needle is bent based on a distance by which the first position information is spaced apart from a straight line on the basis of the second position information and display the calculated result on the screen.

In the (f) displaying, the electrode needle image processor may display the insertion path through which the electrode needle is inserted into the human body within the insertion position range on the screen by expanding a signal reception range to a signal reception range based on the second position information received from the handle-side position sensor, in addition to a signal reception range based on the first position information received from the electrode-side position sensor, and accumulating the first position information and the second position information.

### [Advantageous Effects]

According to the present invention, it is possible to correct electrode needle movement direction information by the bending of the electrode needle, by solving a problem caused by the bending of the electrode needle or limitation of the signal reception range of the position sensor in the electrode device for radio frequency ablation. That is, it is possible to correct a movement direction of the electrode needle inserted into the human body to the site of the lesion, by predicting an angle at which the handle and the electrode needle are bent through the position sensor additionally installed in the handle in addition to the position sensor installed in the end portion of the electrode needle.

Further, it is possible to more widely select the insertion path of the electrode needle into the human body by expanding the signal reception range that was limited to the signal reception range of the position sensor installed in the end portion of the electrode needle, through the position sensor installed in the handle.

Further, if only the position sensor in the end portion of the electrode needle is used, the insertion position may be displayed only in a state in which the electrode needle is very close to the lesion, due to the limitation of the signal reception range, however, according to the present invention, it is possible to confirm the insertion position path that is approachable around the site of the lesion of the body of the patient through the position sensor installed in the handle, thereby more accurately and rapidly coagulating and necrotizing a tissue of the site of the lesion.

### [Description of Drawings]

FIG. 1 is a view schematically illustrating an entire configuration of a device for detecting a position of an electrode inserted into a human body according to an exemplary embodiment of the present invention.
FIGS. 2 to 4 are views schematically illustrating an appearance of the device for detecting a position of an electrode inserted into a human body according to an exemplary embodiment of the present invention.
FIG. 5 is a view illustrating an example in which a position signal is received by expanding a reception range to a reception range of a handle-side position sensor in addition to a reception range of an electrode-side position sensor.
FIG. 6 is an operational flow chart for describing a method for detecting a position of an electrode inserted into a human body according to an exemplary embodiment of the present invention.
FIG. 7 is a view showing an example in which an insertion path through which an electrode needle inserted into a human body will pass is displayed based on first position information and second position information according to an exemplary embodiment of the present invention.
FIG. 8 is a view illustrating an example of recognizing a vertical bending through the electrode-side position sensor and recognizing a lateral bending through the handle-side position sensor according to an exemplary embodiment of the present invention.
FIG. 9 is a diagram illustrating an example in which a guide pipe for matching an axis of the electrode needle with an axis of the electrode-side position sensor is formed.

### [Best Mode]

Hereinafter, exemplary embodiments of the present invention will be described in detail with reference to the accompanying drawings so that those skilled in the art may easily practice the present invention. The present invention may be implemented in various different forms, and is not limited to the exemplary embodiments described herein.

Portions unrelated to the description are omitted in order to obviously describe the present invention, and components that are the same as or similar to each other will be denoted by the same reference numerals throughout the specification

Throughout the present specification, a case in which any one part is "connected" with another part includes a case in which the parts are "directly connected" with each other and a case in which the parts are "electrically connected" with each other with other elements intervening therebetween. In addition, unless explicitly described to the contrary, "comprising" any component will be understood to imply the inclusion of other components rather than the exclusion of any other components.

When it is described that any one part is "on" the other part, it may mean that the part is directly on the other part or any other part is interposed therebetween. On the contrary, when it is described that any one part is "directly on" the other part, there is no other part interposed therebetween.

Terms "first", "second", "third", and the like are used to describe various parts, components, areas, layers, and/or sections, but are not limited thereto. These terms are used only to distinguish one part, component, area, layer, or section from another part, component, area, layer, or section. Accordingly, a first part, a first component, a first area, a first layer, or a first section to be described below may indicate a second part, a second component, a second area, a second layer, or a second section without departing from the scope of the present invention.

Technical terms used herein are merely to describe a specific exemplary embodiment, but are not intended to limit the present invention. Singular forms used herein include plural forms unless context clearly indicates otherwise. A term "comprising" used in the specification specifies a specific characteristic, area, integer, step, operation, element, and/or component, but does not exclude a presence or an addition of any other characteristic, area, integer, step, operation, element, and/or component.

Terms "below", "above", and the like indicating a relative space may be used to more easily describe a relationship between one part illustrated in the drawings with another part. These terms are intended to include other meanings or operations of a device that is being used, in addition to meanings intended in the drawings. For example, when the device in the drawing is inverted, any parts described as being "below" other parts may be described as being "above" the other parts. Therefore, the exemplary term "below" includes both of an upper direction and a lower direction. The device may rotate by 90° or other angles, and the terms indicating a relative space are interpreted according thereto.

Although not defined otherwise, all terms including technical terms and scientific terms used herein have the same meaning as that generally understood by a person having ordinary knowledge in the art to which the present invention pertains. Terms defined in a dictionary generally used are additionally interpreted as having a meaning relevant to the related art documents and contents currently disclosed, and unless defined otherwise, are not interpreted as having an ideal or very official meaning.

Hereinafter, exemplary embodiments of the present invention will be described in detail with reference to the accompanying drawings so as to be easily practiced by a person having ordinary knowledge in the art to which the present invention pertains. However, the present invention may be modified in various different ways and is not limited to the exemplary embodiments described herein.

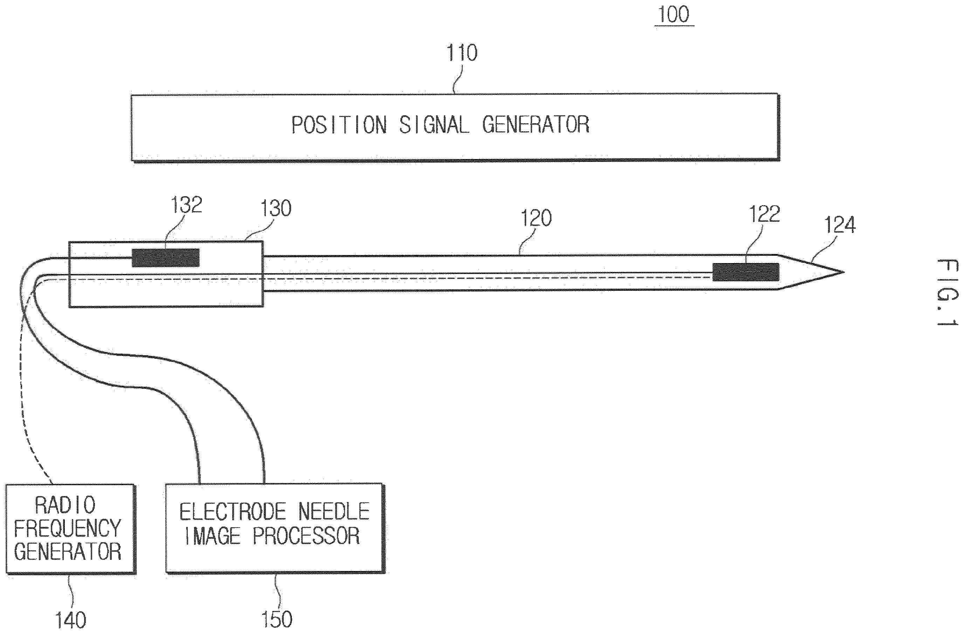

FIG. 1 is a diagram schematically illustrating an entire configuration of a device for detecting a position of an electrode inserted into a human body according to an exemplary embodiment of the present invention.

Referring to FIG. 1, a device 100 for detecting a position of an electrode inserted into a human body according to an exemplary embodiment of the present invention includes a position signal generator 110, an electrode needle 120, a handle 130, a radio frequency generator 140, an electrode needle image processor 150, etc.

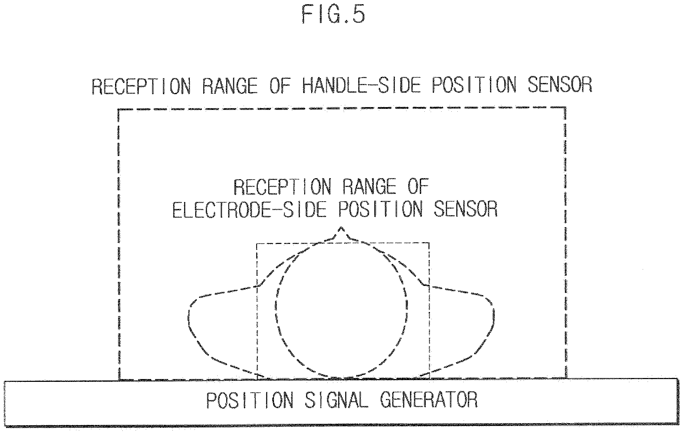

The position signal generator 110 generates a position signal. That is, the position signal generator 110 is installed on a pad or a bed on which a patient is lying to generate a position signal toward the patient as illustrated in FIG. 5. Therefore, a reception range of the generated position signal is determined through an electrode-side position sensor 122 of the electrode needle 120 approaching a site of a lesion of the patient and a handle-side position sensor 132.

The electrode needle 120 has the electrode-side position sensor 122 provided in an interior of a lance needle 124, the electrode-side position sensor 122 receiving the position signal to generate first position information.

The handle 130 is connected to the electrode needle 120 and has the handle-side position sensor 132 provided therein, the handle-side position sensor 132 receiving the position signal to generate second position information.

The radio frequency generator 140 generates a high frequency alternating current and supplies the high frequency alternating current to the electrode needle 120 by selective connection of an active electrode or a passive electrode to a positive terminal and a negative terminal.

The electrode needle image processor 150 receives the first position information from the electrode-side position sensor 122 provided in the electrode needle 120, and the second position information from the handle-side position sensor 132 provided in the handle 130 to display an insertion path on a screen based on the first position information and the second position information when the electrode needle 120 is inserted into the human body.

For example, the electrode needle image processor 150 may be a computerized tomography (CT) machine, a magnetic resonance imaging (MRI) machine, or the like, and may also be implemented by a separate machine interworking with the CT machine, MRI machine, or the like.

Further, the electrode needle image processor 150 displays an insertion position range approachable by the electrode needle 120 around a site of a lesion based on the second position information received from the handle-side position sensor 132, and displays an insertion path through which the electrode needle 120 inserted into the human body will pass, based on the first position information received from the electrode-side position sensor 122 when the electrode needle 120 is inserted into the human body within the insertion position range.

Further, the electrode needle image processor 150 recognizes, based on the first position information and the second position information, that the electrode needle 120 is inserted while being bent in the human body when the insertion path is not a straight line, and calculates an angle and a radius of curvature at which the electrode needle is bent based on a distance by which the first position information is spaced apart from a straight line on the basis of the second position information and displays the calculated result on the screen.

Further, as illustrated in FIG. 5, the electrode needle image processor 150 displays the insertion path through which the electrode needle is inserted into the human body on the screen by expanding a signal reception range to a signal reception range based on the second position information received from the handle-side position sensor 132, in addition to a signal reception range based on the first position information received from the electrode-side position sensor 122, and accumulating the first position information and the second position information.

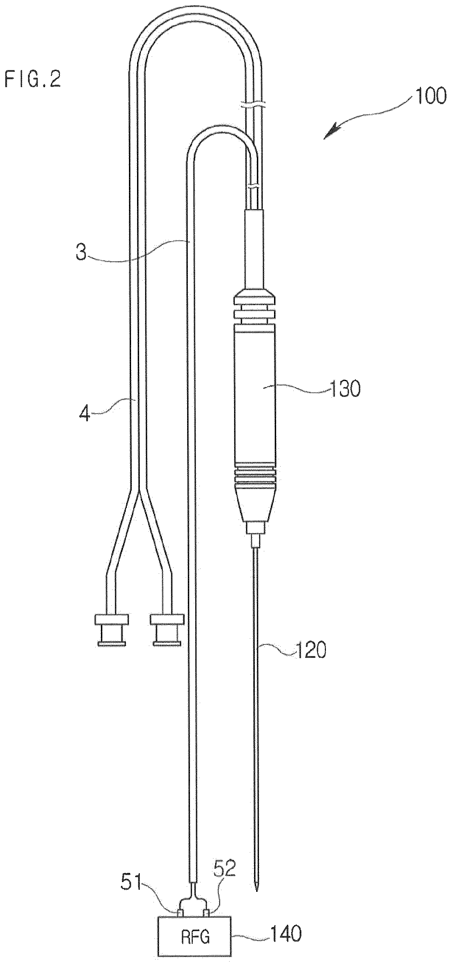

FIGS. 2 to 4 are diagrams schematically illustrating an appearance of the device for detecting a position of an electrode inserted into a human body according to an exemplary embodiment of the present invention.

Referring to FIGS. 2 to 4, the device 100 for detecting a position of an electrode inserted into a human body according to an exemplary embodiment of the present invention further includes an electrode wire 3, a cooling pipe 4, and the radio frequency generator 140, in addition to the aforementioned electrode needle 120 and the handle 130.

As illustrated in FIGS. 2 to 4, the electrode needle 120 includes a body 11 inserted into a tissue of a site of a lesion, and an active electrode 13 and a passive electrode 15 that are wound around the body 11.

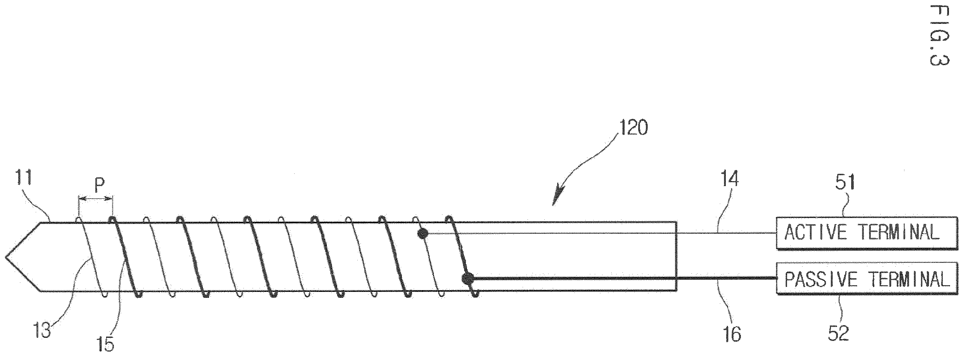

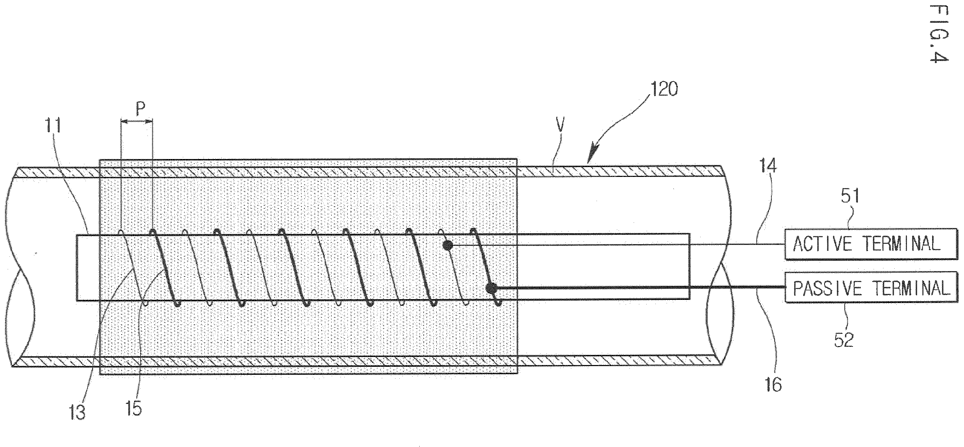

Here, the body 11 is formed in a needle shape that is long and fine as illustrated in FIG. 3, or in a cylindrical shape that is long and fine as illustrated in FIG. 4. In the case in which the body 11 is formed in the needle shape as illustrated in FIG. 3, an end thereof is sharp so as to be easily inserted into a tissue of a site of a lesion, and the other end thereof is connected to the handle as illustrated in FIG. 2. In the case in which the body 11 is formed in the cylindrical shape, when being applied to a catheter, it is connected to an end portion of a moving wire.

The active electrode 13 and the passive electrode 15 radiate a high frequency alternating current generated from the radio frequency generator 140 to the electrode needle 120, and are each inclinedly wound in a spiral direction toward a rear end side from a front end portion of an outer circumferential surface of the body in two or more plural times at the same lead angle while being in parallel with each other.

As illustrated, the active electrode 13 has the other end connected to an active terminal 51 of the radio frequency generator 140 through an active line 14 of the electrode wire 3, and the passive electrode 15 has the other end connected to a passive terminal 52 of the radio frequency generator 140 through a passive line 16 of the electrode wire 3. In the case, the active terminal 51 or the passive terminal 52 may be a positive electrode and may also be a negative electrode as required.

The handle 130 that is a part gripped by an operator when intending to use the electrode needle 120, is disposed at the rear end of the electrode needle 120, and has the electrode wire 3 extended to be long to the radio frequency generator 140 for electrically connecting the electrode needle 120 and the radio frequency generator 140, and the cooling pipe 4 connected thereto for supplying and collecting cooling water to circulate the cooling water.

As illustrated in FIG. 3 and 4, the passive electrode 15 is also inclinedly wound around the electrode needle 120 according to the present invention, in between the active electrode 13 wound in the spiral direction, and the passive electrode 15 and the active electrode 13 maintain an interval therebetween. Therefore, heat generation starts centered at an intermediate point of a pitch P of each electrode 13 and 15, at the time of radiating high frequency energy and in this case, since the pitch P is shorter than a diameter of the body 11, a heat generation range is formed in a cylindrical shape surrounding the body 11, and more preferably, when the pitch P between the electrodes 13 and 15 is constant as in FIGS. 3 and 4, that is, when the interval between the electrodes 13 and 15 alternating each other is constant, the heat generation range has a cylindrical shape having a longitudinal section as illustrated in FIG. 4.

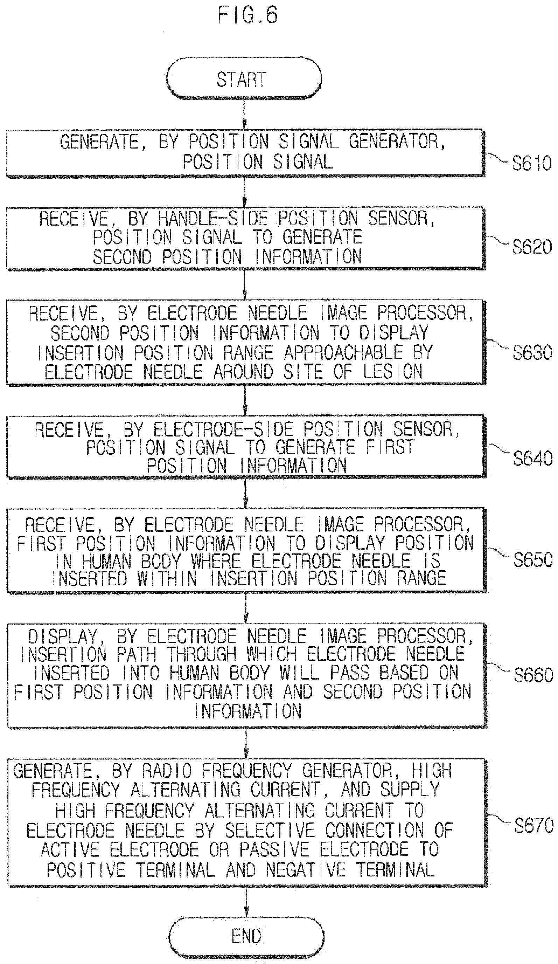

FIG. 6 is an operational flow chart for describing a method for detecting a position of an electrode inserted into a human body according to an exemplary embodiment of the present invention.

Referring to FIG. 6, the device 100 for detecting a position of an electrode inserted into a human body according to an exemplary embodiment of the present invention first generates a position signal in the position signal generator 110 (S610). That is, the position signal generator 110 radiates a position signal toward a human body of a patient laying on a bed or a pad as illustrated in FIG. 5, and accordingly, the position signal is received by the electrode-side position sensor 122 and the handle-side position sensor 132 that are gripped by a hand of the operator within the reception range as in FIG. 5.

Next, the handle-side position sensor 132 provided in the handle 130 receives the position signal to generate second position information (S620). That is, the handle-side position sensor 132 has a reception range wider than that of the electrode-side position sensor 122 as illustrated in FIG. 5, generates the second position information assisting in predicting an insertion path before the electrode needle 120 is inserted into a site of a lesion of a human body, and transmits the generated second position information to the electrode needle image processor 150.

Next, the electrode needle image processor 150 receives the second position information to display an insertion position range approachable by the electrode needle around the site of the lesion as illustrated in FIG. 5 (S630). Therefore, the operator may rapidly recognize the site of the lesion of the patient to insert the electrode needle 120 into the corresponding site of the lesion.

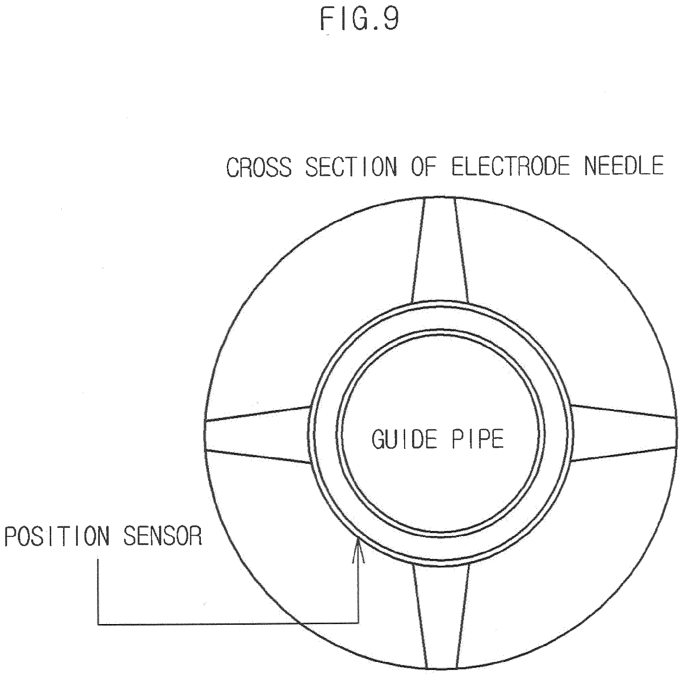

Next, the electrode-side position sensor 122 provided in the electrode needle 120 receives the position signal to generate first position information (S640). Here, the electrode needle 120 interrupts the signal of the position sensor when having a magnetic property due to characteristics of metal material. Therefore, before installing the electrode-side position sensor 122 in the electrode needle 120, an operation of removing the magnetic property of the metallic electrode needs to be performed first. In this case, a guide pipe may be included as illustrated in FIG. 9, in order to match an axis of the electrode needle 120 and an axis of the electrode-side position sensor 122. FIG. 9 is a diagram illustrating an example in which a guide pipe for matching an axis of the electrode needle with an axis of the electrode-side position sensor is formed.

Next, the electrode needle image processor 150 receives the first position information from the electrode-side position sensor 122 to display a position in the human body where the electrode needle 120 is inserted within the insertion position range (S650).

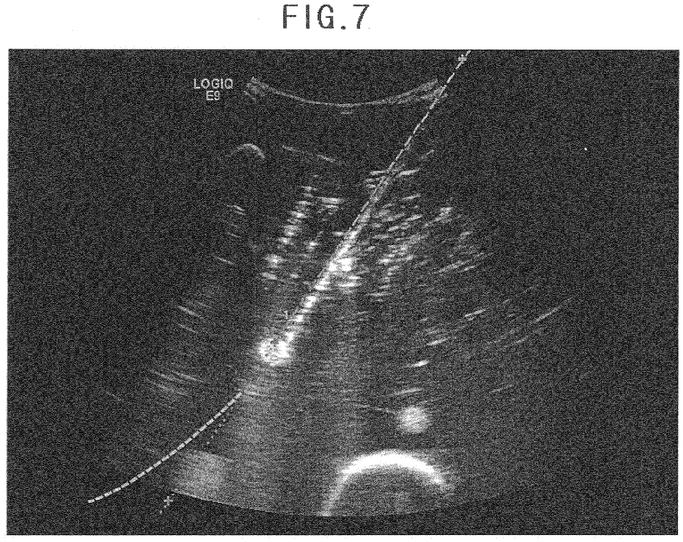

Next, the electrode needle image processor 150 displays the insertion path through which the electrode needle inserted into the human body will pass on a screen based on the first position information and the second position information as illustrated in FIG. 7 (S660). FIG. 7 is a view showing an example in which an insertion path through which an electrode needle inserted into a human body will pass is displayed based on first position information and second position information according to an exemplary embodiment of the present invention. As illustrated in FIG. 7, the electrode needle image processor 150 displays the insertion path in a straight line or a curved line on the screen based on the first position information and the second position information.

Further, the electrode needle image processor 150 recognizes, based on the first position information and the second position information, that the electrode needle is inserted while being bent in the human body when the insertion path is not a straight line, and calculates an angle and a radius of curvature at which the electrode needle is bent based on a distance by which the first position information is spaced apart from a straight line on the basis of the second position information and displays the calculated result on the screen.

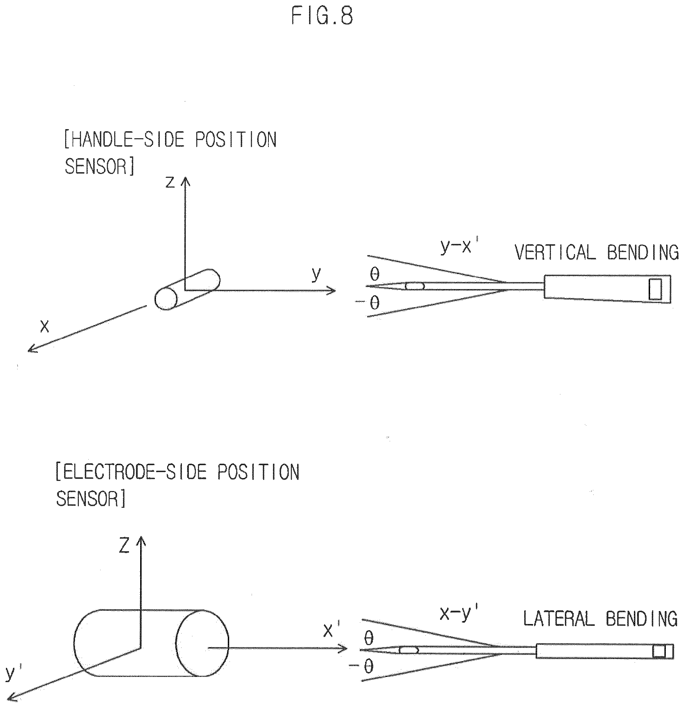

Here, the electrode needle image processor 150 may display a curved insertion path as illustrated in FIG. 7, by recognizing a vertical bending as illustrated in FIG. 8 based on the first position information received from the electrode-side position sensor 122 when the electrode needle is inserted into the human body. FIG. 8 is a view illustrating an example of recognizing a vertical bending through the electrode-side position sensor and recognizing a lateral bending through the handle-side position sensor according to an exemplary embodiment of the present invention. Further, the electrode needle image processor 150 may display a curved insertion path as illustrated in FIG. 7, by recognizing a lateral bending as illustrated in FIG. 8 based on the second position information received from the handle-side position sensor 132.

Further, the electrode needle image processor 150 displays the insertion path through which the electrode needle is inserted into the human body and within the insertion position range on the screen as illustrated in FIG. 7 by expanding a signal reception range to the signal reception range based on the second position information received from the handle-side position sensor 132, in addition to the signal reception range based on the first position information received from the electrode-side position sensor 122, and accumulating the first position information and the second position information.

Next, the radio frequency generator 140 generates a high frequency alternating current and supplies the high frequency alternating current to the electrode needle by selective connection of the active electrode or the passive electrode to a positive terminal and a negative terminal (S670).

That is, the high frequency alternating current is radiated between the active electrode 13 and the passive electrode 15 by the operation of the radio frequency generator 140 and at this point, in the case of the exemplary embodiment illustrated in FIGS. 3 and 4, the high frequency energy is radiated between every adjacent active electrode 13 and passive electrode 15 at a pitch P interval as mentioned above, such that a cylindrical high frequency energy radiation area is formed all over as indicated by A. Therefore, the two electrodes 13 and 15 generate a frictional heat, as an ion in the tissue of the site of the lesion vibrates by the energy generated in the radiation area, and a temperature of the tissue of the site of the lesion is increased by the heat, such that it is possible to effectively perform the operation on the site of the lesion at a minimum thickness, that is, without causing damage to other adjacent tissues, by the cylindrical radiation area following a shape of the site of the lesion.

According to the present invention as described above, it is possible to implement a device and a method for detecting a position of an electrode inserted into a human body that predict a path through a position sensor in a handle part before the electrode is inserted and display the path in order to accurately place an electrode needle on a lesion through a position sensor in the electrode needle after the electrode is inserted into the human body during ultrasonic image diagnosis, computerized tomography (CT), or magnetic resonance imaging (MRI) guide radio frequency ablation (RFA) operation in an electrode device for radio frequency ablation that cauterizes and necrotizes a site of a lesion, such as a tumor tissue of a body organ, etc., by heating the site of the lesion with high frequency.

Those skilled in the art to which the present invention pertains should understand that since the present invention may be practiced in different specific forms without changing the technical spirit or essential feature of the present invention, the above-mentioned exemplary embodiments are not restrictive but are exemplary in all aspects. It should be interpreted that the scope of the present invention is defined by the following claims rather than the detailed description and all modifications or alterations deduced from the meaning, the scope, and equivalences of the claims are included in the scope of the present invention.

### [Industrial Applicability]

The present invention may be applied to a device and a method for detecting a position of an electrode inserted into a human body that predict a path through a position sensor in a handle part before the electrode is inserted and display the path in order to accurately place an electrode needle on a lesion through a position sensor in the electrode needle after the electrode is inserted into the human body in an electrode device for radio frequency ablation.

## Claims

1. A device for detecting a position of an electrode inserted into a human body, comprising:
a position signal generator generating a position signal;
an electrode needle having an electrode-side position sensor that is provided in an interior of a lance needle thereof and receives the position signal to generate first position information; and
a handle connected to the electrode needle and having a handle-side position sensor that is included therein and receives the position signal to generate second position information.

2. The device of claim 1, wherein the electrode needle includes a body inserted into a tissue of a site of a lesion, and an active electrode and a passive electrode that are wound around the body.

3. The device of claim 2, wherein the body is formed in a needle shape that is long and fine, or in a cylindrical shape that is long and fine, the body formed in the needle shape has an end that is sharp so as to be easily inserted into the tissue of the site of the lesion and the other end that is connected to the handle, and the body formed in the cylindrical shape is connected to an end portion of a moving wire when being applied to a catheter.

4. The device of claim 2, further comprising a radio frequency generator generating a high frequency alternating current and supplying the high frequency alternating current to the electrode needle by selective connection of the active electrode or the passive electrode to a positive terminal and a negative terminal.

5. The device of claim 4, wherein the active electrode and the passive electrode radiate the high frequency alternating current generated from the radio frequency generator to the electrode needle, and are each inclinedly wound in a spiral direction toward a rear end side from a front end portion of an outer circumferential surface of the body in two or more plural times at the same lead angle while being in parallel with each other.

6. The device of claim 4, wherein the handle is a part gripped by an operator when intending to use the electrode needle, is disposed at a rear end of the electrode needle, and has an electrode wire extended to be long to the radio frequency generator for electrically connecting the electrode needle and the radio frequency generator, and a cooling pipe connected thereto for supplying and collecting cooling water to circulate the cooling water.

7. The device of claim 1, further comprising an electrode needle image processor receiving the first position information from the electrode-side position sensor provided in the electrode needle, and the second position information from the handle-side position sensor provided in the handle to display an insertion path on a screen based on the first position information and the second position information when the electrode needle is inserted into the human body.

8. The device of claim 7, wherein the electrode needle image processor displays an insertion position range approachable by the electrode needle around a site of a lesion based on the second position information received from the handle-side position sensor, and displays the insertion path through which the electrode needle inserted into the human body will pass, based on the first position information received from the electrode-side position sensor when the electrode needle is inserted into the human body within the insertion position range.

9. The device of claim 7, wherein the electrode needle image processor recognizes, based on the first position information and the second position information, that the electrode needle is inserted while being bent in the human body when the insertion path is not a straight line, and calculates an angle and a radius of curvature at which the electrode needle is bent based on a distance by which the first position information is spaced apart from a straight line on the basis of the second position information and displays the calculated result on the screen.

10. The device of claim 7, wherein the electrode needle image processor displays the insertion path through which the electrode needle is inserted into the human body on the screen by expanding a signal reception range to a signal reception range based on the second position information received from the handle-side position sensor, in addition to a signal reception range based on the first position information received from the electrode-side position sensor, and accumulating the first position information and the second position information.

11. A method for detecting a position of an electrode inserted into a human body, comprising:
(a) generating, by a position signal generator, a position signal;
(b) receiving, by a handle-side position sensor, the position signal to generate second position information;
(c) receiving, by an electrode needle image processor, the second position information to display an insertion position range approachable by an electrode needle around a site of a lesion;
(d) receiving, by an electrode-side position sensor, the position signal to generate first position information;
(e) receiving, by the electrode needle image processor, the first position information to display a position in the human body where the electrode needle is inserted within the insertion position range; and
(f) displaying, by the electrode needle image processor, an insertion path through which the electrode needle inserted into the human body will pass, on a screen based on the first position information and the second position information.

12. The method of claim 11, further comprising: (g) generating, by a radio frequency generator, a high frequency alternating current and supplying the high frequency alternating current to the electrode needle by selective connection of an active electrode or a passive electrode to a positive terminal and a negative terminal.

13. The method of claim 11, wherein in the (f) displaying, the electrode needle image processor displays the insertion path in a straight line or a curved line on the screen based on the first position information and the second position information.

14. The method of claim 11, wherein in the (f) displaying, the electrode needle image processor recognizes, based on the first position information and the second position information, that the electrode needle is inserted while being bent in the human body when the insertion path is not a straight line, and calculates an angle and a radius of curvature at which the electrode needle is bent based on a distance by which the first position information is spaced apart from a straight line on the basis of the second position information and displays the calculated result on the screen.

15. The method of claim 11, wherein in the (f) displaying, the electrode needle image processor displays the insertion path through which the electrode needle is inserted into the human body within the insertion position range on the screen by expanding a signal reception range to a signal reception range based on the second position information received from the handle-side position sensor, in addition to a signal reception range based on the first position information received from the electrode-side position sensor, and accumulating the first position information and the second position information.
